Europäisches Patentamt

European Patent Office

Office européen des brevets

(19)

(11) EP 0 950 720 A1

(12) **EUROPEAN PATENT APPLICATION**

(43) Date of publication:
20.10.1999 Bulletin 1999/42

(51) Int Cl.⁶: **C12Q 1/68**

(21) Application number: **99250122.1**

(22) Date of filing: **15.04.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **15.04.1998 US 60922**

(71) Applicant: **Affymetrix, Inc.**
**Santa Clara, CA 95051 (US)**

(72) Inventors:
• **Lipschutz, Robert J.**
**Palo Alto, CA 94301 (US)**
• **Fodor, Stephen**
**Palo Alto, CA 94301 (US)**

(74) Representative: **UEXKÜLL & STOLBERG**
**Patentanwälte**
**Beselerstrasse 4**
**22607 Hamburg (DE)**

(54) **Methods for polymorphism identification and profiling**

(57)    The invention provides methods of using probe arrays for polymorphism identification and profiling. Such methods entail constructing a first array of probes that span and are complementary to one or more known DNA sequences. This array is hybridized with nucleic acid samples from different individuals to identify a collection of polymorphisms. A second array is then constructed to determine a polymorphic profile of an individual at the collection of polymorphic sites. The polymorphic profile is useful for, e.g., genetic mapping, epidemiology, diagnosis and forensics.

EP 0 950 720 A1

**Description**

TECHNICAL FIELD

The invention resides in the technical fields of molecular genetics, medicine and forensics.

BACKGROUND

The genomes of all organisms undergo spontaneous mutation in the course of their continuing evolution generating variant forms of progenitor sequences (Gusella, *Ann. Rev. Biochem.* 55, 831-854 (1986)). The variant form may confer an evolutionary advantage or disadvantage relative to a progenitor form or may be neutral. In some instances, a variant form confers a lethal disadvantage and is not transmitted to subsequent generations of the organism. In other instances, a variant form confers an evolutionary advantage to the species and is eventually incorporated into the DNA of many or most members of the species and effectively becomes the progenitor form. In many instances, both progenitor and variant form(s) survive and co-exist in a species population. The coexistence of multiple forms of a sequence gives rise to polymorphisms.

Several different types of polymorphism have been reported. A restriction fragment length polymorphism (RFLP) means a variation in DNA sequence that alters the length of a restriction fragment as described in Botstein et al., *Am. J. Hum. Genet.* 32, 314-331 (1980). Other polymorphisms take the form of short tandem repeats (STRs) that include tandem di-, tri- and tetra-nucleotide repeated motifs. Some polymorphisms take the form of single nucleotide variations between individuals of the same species. Such polymorphisms are far more frequent than RFLPs, STRs and VNTRs. Single nucleotide polymorphisms can occur anywhere in protein-coding sequences, intronic sequences, regulatory sequences, or intergenomic regions.

Many polymorphisms probably have little or no phenotypic effect. Some polymorphisms, principally those occurring within coding sequences, are known to be the direct cause of serious genetic diseases, such as sickle cell anemia. Polymorphisms occurring within a coding sequence typically exert their phenotypic effect by leading to a truncated or altered expression product. Still other polymorphisms, particularly those in promoter regions and other regulatory sequences, may influence a range of disease-susceptibility, behavioral and other phenotypic traits through their effect on gene expression levels. That is, such polymorphisms may lead to increased or decreased levels of gene expression without necessarily affecting the nature of the expression product.

Some methods for detecting polymorphisms using arrays of oligonucleotides are described in WO 95/11995 (incorporated by reference in its entirety for all purposes). Some such arrays include four probe sets. A first probe set includes overlapping probes spanning a region of interest in a reference sequence. Each probe in the first probe set has an interrogation position that corresponds to a nucleotide in the reference sequence. That is, the interrogation position is aligned with the corresponding nucleotide in the reference sequence, when the probe and reference sequence are aligned to maximize complementarily between the two. For each probe in the first set, there are three corresponding probes from three additional probe sets. Thus, there are four probes corresponding to each nucleotide in the reference sequence. The probes from the three additional probe sets are identical to the corresponding probe from the first probe set except at the interrogation position, which occurs in the same position in each of the four corresponding probes from the four probe sets, and is occupied by a different nucleotide in the four probe sets.

Such an array is hybridized to a labelled target sequence, which may be the same as the reference sequence, or a variant thereof. The identity of any nucleotide of interest in the target sequence can be determined by comparing the hybridization intensities of the four probes having interrogation positions aligned with that nucleotide. The nucleotide in the target sequence is the complement of the nucleotide occupying the interrogation position of the probe with the highest hybridization intensity.

WO 95/11995 also describes subarrays that are optimized for detection of a variant forms of a precharacterized polymorphism. A subarray contains probes designed to be complementary to a second reference sequence, which can be an allelic variant of the first reference sequence. The second group of probes is designed by the same principles as above except that the probes exhibit complementarity to the second reference sequence. The inclusion of a second group can be particular useful for analyzing short subsequences of the primary reference sequence in which multiple mutations are expected to occur within a short distance commensurate with the length of the probes (i.e., two or more mutations within 9 to 21 bases).

A further strategy for detecting a polymorphism using an array of probes is described in EP 717,113. In this strategy, an array contains overlapping probes spanning a region of interest in a reference sequence. The array is hybridized to a labelled target sequence, which may be the same as the reference sequence or a variant thereof. If the target sequence is a variant of the reference sequence, probes overlapping the site of variation show reduced hybridization intensity relative to other probes in the array. In arrays in which the probes are arranged in an ordered fashion stepping through the reference sequence (e.g., each successive probe has one fewer 5' base and one more 3' base than its predecessor), the loss of hybridization intensity is manifested as a "footprint" of probes approximately centered about the point of variation between the target sequence and reference sequence.

SUMMARY OF THE CLAIMED INVENTION

In one aspect, the invention provides methods of polymorphism analysis. Such methods entail constructing a first array of probes for polymorphism identification. The probes in such an array span and are complementary to one or more known DNA sequences. The first array of probes is then hybridized with nucleic acid samples from different individuals. Differences in the hybridization pattern of the samples to the probes among the different individuals indicate the location of one or more polymorphic sites in the one or more DNA sequences. The above steps are repeated, as needed, until a collection of polymorphic sites in known DNA sequences has been identified. A second array of probes is then constructed for polymorphism profiling. The second array comprises a first set of probes spanning each of the polymorphic sites in the collection and complementary to polymorphic forms present in the known sequences, and a second set of probes spanning each of the polymorphic sites in the collection and complementary to polymorphic forms absent in the known DNA sequences. The second array of probes is hybridized to a nucleic acid sample from a further individual. The hybridization intensities of probes in the first and second sets of probes are analyzed to determine a profile of polymorphic forms present in the further individual. In some methods, the further individual has a known characteristic whose presence or absence is unknown in the different individuals used in the prior polymorphism analysis. This characteristic can be, for example, the presence of absence of a disease or of being suspected of perpetrating a crime.

Some methods further comprises retrieving a known DNA sequences from a computer database for use in the polymorphism identification steps. The probes in the first array are selected to be complementary to the known sequence. Often, the known sequences used for polymorphism identification are of unknown function. In some methods, the sequences used for polymorphism identification are expressed sequence tags. In some methods, at least 100 known sequences are used for polymorphism identification. In some methods, at least some of the known sequences are unlinked; for example, known sequences can occur on at least 2 chromosomes, or each of the 23 human chromosomes.

In some methods, the further nucleic acid sample is RNA or cDNA. In such methods, the hybridization intensities of probes in the second array can be used to identify a subset of polymorphic sites in a subset of known sequences which are expressed in the further nucleic acid sample. Profiles of the subset of polymorphic sites can readily be obtained from an RNA sample without amplification.

In another aspect, the invention provides methods of determining whether discrepancies in published sequences represent true genetic variation or are sequencing errors. Such methods entail retrieving multiple versions of a nucleic acid sequence from a published source. The multiple versions to identify point(s) of diversion. An array of probes is then designed that span and are complementary to part(s) of the nucleic acid sequence spanning the point(s) of diversion. Nucleic acid samples from multiple individuals are then hybridized to the array. The existence of difference(s) in the hybridization intensity of probes spanning a point of diversion among the individuals indicates a polymorphism at the point of diversion, and lack of difference in hybridization intensity of probes spanning a point of diversion among the individuals indicates the point of diversion was due to a sequencing error. In some methods, the published sources of sequences is a computer database. In some methods, the multiple version of the nucleic acid sequence are retrieved as trace profiles.

In another aspect, the invention provides methods of polymorphism profiling. Such methods entail providing an array of immobilized probes. The array comprises a first set of probes spanning each of a collection of polymorphic sites in known sequences and complementary to a first allelic forms of the sites, and a second set of probes spanning each of the polymorphic sites in the collection and complementary to second allelic forms of the sites. The collection of polymorphic sites includes at least 10 unlinked polymorphic sites. A nucleic acid sample from an individual is hybridized to the array of probes and the hybridization intensities of probes in the first and second probe sets are analyzed to determine a profile of polymorphic forms present in the individual. In some such methods, the collection of polymorphic sites includes a polymorphic site on each of the 23 human chromosomes. In some methods, the collection of polymorphic sites includes at least 100 polymorphic sites.

In some methods, the hybridizing step is repeated for nucleic acid samples from a population of individuals, each of whom is characterized for the presence or absence of a phenotype, to determine a profile of polymorphic forms present in each individual in the population, and the method further comprises correlating profiles of polymorphic forms with the presence or absence of the phenotype in the population.

In some methods, the nucleic acid sample is RNA or cDNA. In such methods, optionally, one can enrich for transcripts of the known sequences in the nucleic acid sample by contacting the nucleic acid sample with probes complementary to the transcripts, whereby the probes hybridize to the transcripts to form complexes, isolating the complexes and dissociating the transcripts of the known sequences from the probes.

DEFINITIONS

A nucleic acid is a deoxyribonucleotide or ribonucleotide polymer in either single-or double-stranded form, including known analogs of natural nucleotides unless otherwise indicated.

An oligonucleotide is a single-stranded nucleic acid ranging in length from 2 to about 500 bases.

A probe is an oligonucleotide capable of binding to a target nucleic acid of complementary sequence through one or more types of chemical bonds, usually through complementary base pairing, usually through hydrogen bond formation. An oligonucleotide probe may include natural (*i.e.* A, G, C, or T) or modified bases (e.g., 7-deazaguanosine, inosine). In addition, the bases in oligonucleotide probe may be joined by a linkage other than a phosphodiester bond, so long as it does not interfere with hybridization. Thus, oligonucleotide probes may be peptide nucleic acids in which the constituent bases are joined by peptide bonds rather than phosphodiester linkages.

Specific hybridization refers to the binding, duplexing, or hybridizing of a molecule only to a particular nucleotide sequence under stringent conditions when that sequence is present in a complex mixture (*e.g.*, total cellular) DNA or RNA. Stringent conditions are conditions under which a probe will hybridize to its target subsequence, but to no other sequences. Stringent conditions are sequencedependent and are different in different circumstances. Longer sequences hybridize specifically at higher temperatures. Generally, stringent conditions are selected to be about 5°C lower than the thermal melting point (Tm) for the specific sequence at a defined ionic strength and pH. The Tm is the temperature (under defined ionic strength, pH, and nucleic acid concentration) at which 50% of the probes complementary to the target sequence hybridize to the target sequence at equilibrium. (As the target sequences are generally present in excess, at Tm, 50% of the probes are occupied at equilibrium). Typically, stringent conditions include a salt concentration of at least about 0.01 to 1.0 M Na ion concentration (or other salts) at pH 7.0 to 8.3 and the temperature is at least about 30°C for short probes (*e.g.*, 10 to 50 nucleotides). Stringent conditions can also be achieved with the addition of destabilizing agents such as formamide. For example, conditions of 5X SSPE (750 mM NaCl, 50 mM Na-Phosphate, 5 mM EDTA, pH 7.4) and a temperature of 25-30°C are suitable for allele-specific probe hybridizations.

A perfectly matched probe has a sequence perfectly complementary to a particular target sequence. The test probe is typically perfectly complementary to a portion (subsequence) of the target sequence. The term "mismatch probe" refer to probes whose sequence is deliberately selected not to be perfectly complementary to a particular target sequence. Although the mismatch(s) may be located anywhere in the mismatch probe, terminal mismatches are less desirable as a terminal mismatch is less likely to prevent hybridization of the target sequence. Thus, probes are often designed to have the mismatch located at or near the center of the probe such that the mismatch is most likely to destabilize the duplex with the target sequence under the test hybridization conditions.

A polymorphic marker or site is the locus at which divergence occurs. Preferred markers have at least two alleles, each occurring at frequency of greater than 1%, and more preferably greater than 10% or 20% of a selected population. A polymorphic locus may be as small as one base pair. Polymorphic markers include restriction fragment length polymorphisms, variable number of tandem repeats (VNTR's), hypervariable regions, minisatellites, dinucleotide repeats, trinucleotide repeats, tetranucleotide repeats, simple sequence repeats, and insertion elements such as Alu. The first identified allelic form is arbitrarily designated as a the reference form and other allelic forms are designated as alternative or variant alleles. The allelic form occurring most frequently in a selected population is sometimes referred to as the wildtype form. Diploid organisms may be homozygous or heterozygous for allelic forms. A diallelic polymorphism has two forms. A triallelic polymorphism has three forms.

A single nucleotide polymorphism (SNP) occurs at a polymorphic site occupied by a single nucleotide, which is the site of variation between allelic sequences. The site is usually preceded by and followed by highly conserved sequences of the allele (e.g., sequences that vary in less than 1/100 or 1/1000 members of the populations).

A single nucleotide polymorphism usually arises due to substitution of one nucleotide for another at the polymorphic site. A transition is the replacement of one purine by another purine or one pyrimidine by another pyrimidine. A transversion is the replacement of a purine by a pyrimidine or vice versa. Single nucleotide polymorphisms can also arise from a deletion of a nucleotide or an insertion of a nucleotide relative to a reference allele.

DETAILED DISCLOSURE

I. General

The invention employs arrays of oligonucleotide probes for de novo identification of polymorphisms and for the use of such polymorphisms in determining a polymorphic profile of an individual. De novo identification of polymorphisms starts with a nucleic acid fragment whose sequence is known, which is designated a reference sequence. The reference sequence can be obtained from a computer database or from the published literature or can be determined by any conventional means. A probe array is constructed containing probes spanning and complementary to the reference sequence, or any segment of interest thereof. The array of probes is hybridized to nucleic acid samples from a collection of individuals. If different allelic forms of the reference sequence are present in the collection of individuals, the array of probes shows a different hybridization pattern to different samples. The hybridization patterns can be interpreted to reveal the location of a polymorphic site in the reference sequence, and in some instances, the polymorphic forms present at this site.

The more individuals that are screened, the more likely it is that a polymorphic site is identified. About ten individuals is sufficient to identify most polymorphic sites. Typically the individuals are unrelated. The individuals need not be from any geographic, religious or ethnic subclass. Indeed, selecting individuals from different subclasses can increase the probability of identifying a polymorphic site. In most instances, the individuals are humans, but plants and animals can also be used. Typically, the individuals have not been characterized for the presence or absence of a selected trait, such as a particular disease.

The above identification process can be carried out on a large scale. For example, the same support can have attached multiple subsets of probes that span and are complementary to multiple reference sequences. The reference sequences need not be related; for example, they can be from different chromosomes. The hybridization pattern of each subset of probes to nucleic acid samples from different individuals can be interpreted independently to determine the existence and nature of polymorphic sites in each of the reference sequences. Alternatively, or additionally, subsets of probes spanning and complementarity to different reference sequences can be immobilized on separate supports, and the supports individually hybridized to samples from different individuals. Ultimately, a collection of polymorphism in a collection of reference sequences is identified. A collection of several thousand polymorphisms identified by this approach is described in commonly owned applications USSN 08/813,159, 60/042,125, 60/050,594.

A secondary array is then constructed to use the previously identified polymorphisms for polymorphic profiling. The secondary array includes a first group of probes, which span polymorphic sites and flanking bases, and are complementary to the reference sequences. The secondary array includes a second group of probes, which also span the polymorphic sites and flanking bases, but which are designed to be complementary to allelic variant forms of the reference sequences. The secondary array typically includes probes spanning a large collection of polymorphic sites (e.g., 1000 or more). The secondary array is hybridized to a nucleic acid sample from a further individual. Analysis of the hybridization pattern indicates which allelic form is present at the polymorphic sites included in the secondary array, thereby developing a polymorphic profile of the individual.

The polymorphic profile can be used in association studies. That is, by determining polymorphic individuals in a population of individuals, each of whom has been characterized for the presence or absence of a phenotypic trait, one can determine which polymorphic forms, alone or in combination, are correlated with the trait. Alternatively, once a correlation of traits with polymorphic forms has been performed, determination of a polymorphic profile in an individual can be used to predict susceptibility to traits without direct phenotypic testing of the individual. Polymorphic profiles are also useful in forensics and paternity testing.

## 2. Reference Sequences

Reference sequences for polymorphic site identification are often obtained from computer databases such as Genbank, the Stanford Genome Center, The Institute for Genome Research and the Whitehead Institute. The latter databases are available at http://www-genome.wi.mit.edu; http://shgc.stanford.edu and http://ww.tigr.org. A reference sequence can vary in length from 5 bases to at least 100,000 bases. References sequences are typically of the order of 100-1000 bases. The reference sequence can be from expressed or nonexpressed regions of the genome. In some methods, in which RNA samples are used, highly expressed reference sequences are sometimes preferred to avoid the need for RNA amplification. The reference sequences can be from proximate areas of the genome or can be unrelated. Preferably, diverse reference sequences are analyzed to identify a correspondingly diverse collection of polymorphisms. For example, the reference sequences can come from two or more chromosomes of the human genome. In some methods, reference sequences from each of the 23 human chromosomes is present. In some instances, the function of a reference sequence is known, but more commonly reference sequences are of unknown function. reference sequences can also be from episomes such as mitochondrial DNA.

## 3. Nucleic Acid Sample Preparation

The nucleic acid samples hybridized to arrays can be genomic, RNA or cDNA. Genomic DNA samples are usually subject o amplification before application to an array. An individual genomic DNA segment from the same genomic location as a designated reference sequence can be amplified by using primers flanking the reference sequence. Multiple genomic segments corresponding to multiple reference sequences can be prepared by multiplex amplification including primer pairs flanking each reference sequence in the amplification mix. Alternatively, the entire genome can be amplified using random primers (typically hexamers) (see Barrett et al., *Nucleic Acids Research* 23, 3488-3492 (1995)) or by fragmentation and reassembly (see, e.g., Stemmer et al., *Gene* 164, 49-53 (1995)). Genomic DNA can be obtained from virtually any tissue source (other than pure red blood cells). For example, convenient tissue samples include whole blood, semen, saliva, tears, urine, fecal material, sweat, buccal, skin and hair.

RNA samples are also often subject to amplification. In this case amplification is typically preceded by reverse transcription. Amplification of all expressed mRNA can be performed as described by commonly owned WO 96/14839 and WO 97/01603. Selective amplification of transcripts containing polymorphic site contained on a chip can be achieved by the same approach as for genomic DNA. Selected transcripts containing the polymorphic sites tiled on a substrate can also be enriched by prehybridizing with biotin-labelled oligonucleotides complementarity to such transcripts. After separation of unbound oligonucleotides, hybridization complexes can be separated by affinity chromatography to streptavidin-coated magnetic beads. If RNA species are in molar excess relative to corresponding labelled oligonucleotides, and different oligonucleotides are used in equimolar amounts, the prehybridization step also has the effect of equalizing the concentrations of the RNA species for which probes are included in the array.

In some methods, in which arrays are designed to tile polymorphic sites occurring in highly expressed sequences, amplification of RNA is unnecessary. A species occurring at a relative abundance of 1:30,000 in RNA of total concentration of 0.1 mg/ml can be detected. The choice of tissue from which the sample is obtained affects the relative and absolute levels of different RNA transcripts in the sample. For example, cytochromes P450 are expressed at high levels in the liver.

### 4. Methods of amplification

The PCR method of amplification is described in *PCR Technology: Principles and Applications for DNA Amplification* (ed. H.A. Erlich, Freeman Press, NY, NY, 1992); *PCR Protocols: A Guide to Methods and Applications* (eds. Innis, et al., Academic Press, San Diego, CA, 1990); Mattila et al., *Nucleic Acids Res.* 19, 4967 (1991); Eckert et al., *PCR Methods and Applications* 1, 17 (1991); *PCR* (eds. McPherson et al., IRL Press, Oxford); and U.S. Patent 4,683,202 (each of which is incorporated by reference for all purposes). Nucleic acids in a target sample are usually labelled in the course of amplification by inclusion of one or more labelled nucleotides in the amplification mix. Labels can also be attached to amplification products after amplification e.g., by end-labelling. The amplification product can be RNA or DNA depending on the enzyme and substrates used in the amplification reaction.

Other suitable amplification methods include the ligase chain reaction (LCR) (see Wu and Wallace, *Genomics* 4, 560 (1989), Landegren et al., *Science* 241, 1077 (1988), transcription amplification (Kwoh et al., *Proc. Natl. Acad. Sci. USA* 86, 1173 (1989)), and self-sustained sequence replication (Guatelli et al., *Proc. Nat. Acad. Sci. USA,* 87, 1874 (1990)) and nucleic acid based sequence amplification (NASBA). The latter two amplification methods involve isothermal reactions based on isothermal transcription, which produce both single stranded RNA (ssRNA) and double stranded DNA (dsDNA) as the amplification products in a ratio of about 30 or 100 to 1, respectively.

### 5. Probe Arrays

The primary arrays of probes contain at least a first set of probes that tiles one or more reference sequences (or regions of interest therein). Tiling means that the probe set contains overlapping probes which are complementary to and span a region of interest in the reference allele. For example, a probe set might contain a ladder of probes, each of which differs from its predecessor in the omission of a 5' base and the acquisition of an additional 3' base. The probes in a probe set may or may not be the same length. The number of probes can vary widely from about 5, 10, 20, 50, 100, 1000, to 10,000 or 100,000.

Such an array is hybridized to target samples from individuals under test and/or to a control sample known to contain the reference sequence(s) tiled by the array. Optionally, the array can by hybridized simultaneously to more than one target sample or to a target sample and reference sequence by use of two-color labelling (e.g., the reference sequence bears one label and a target sample bears a second label). If the array is hybridized to a control reference sequence (or a target sequence that is identical to the reference sequence), all probes in the first probe set specifically hybridize to the reference sequence. If the array is hybridized to a target sample containing a target sequence that differs from the reference sequence at a polymorphic site, then probes flanking the polymorphic site do not show specific hybridization, whereas other probes in the first probe set distal to the polymorphic site do show specific hybridization. The existence of a polymorphism is also manifested by differences in normalized hybridization intensities of probes flanking the polymorphism when the probes hybridized to corresponding targets from different individuals. For example, relative loss of hybridization intensity in a "footprint" of probes flanking a polymorphism signals a difference between the target and reference (i.e., a polymorphism) (see EP 717,113, incorporated by reference in its entirety for all purposes). Additionally, hybridization intensities for corresponding targets from different individuals can be classified into groups or clusters suggested by the data, not defined *a priori,* such that isolates in a give cluster tend to be similar and isolates in different clusters tend to be dissimilar. See WO 97/29212 (incorporated by reference in its entirety for all purposes).

Optionally, primary arrays of probes can also contain second, third and fourth probe sets as described in WO 95/11995. The probes from the three additional probe sets are identical to a corresponding probe from the first probe set except at the interrogation position, which occurs in the same position in each of the four corresponding probes from the four probe sets, and is occupied by a different nucleotide in the four probe sets. After hybridization of such an array to a labelled target sequence, analysis of the pattern of label revealed the nature and position of differences between the target and reference sequence. For example, comparison of the intensities of four corresponding probes reveals the identity of a corresponding nucleotide in the target sequences aligned with the interrogation position of the probes. The corresponding nucleotide is the complement of the nucleotide occupying the interrogation position of the probe showing the highest intensity.

Optionally, primary arrays tile both strands of reference sequences. Both strands are tiled separately using the same principles described above, and the hybridization patterns of the two tilings are analyzed separately. Typically, the hybridization patterns of the two strands indicates the same results (i.e., location and/or nature of polymorphic form) increasing confidence in the analysis. Occasionally, there may be an apparent inconsistency between the hybridization patterns of the two strands due to, for example, base-composition effects on hybridization intensities. Such inconsistency signals the desirability of rechecking a target sample either by the same means or by some other sequencing methods, such as use of an ABI sequencer.

The secondary arrays used for analyzing previously identified polymorphisms typically differ from the primary arrays in the following respects. First, whereas probes are typically included to span the entire length of a reference sequence in primary arrays, in secondary arrays only a segment of a reference sequence containing a polymorphic site and immediately flanking bases is typically spanned in secondary arrays. For example, this segment is often of a length commensurate with that of the probes. Second, a secondary array typically includes at least two groups of probes. A first group of probes is designed based on the reference sequence, and the second group based on a polymorphic form thereof. If there are three polymorphic forms at a given polymorphic site, a third group of probes can be included. Finally, because fewer probes are generally required to analyze precharacterized polymorphisms than in the de novo identification of polymorphisms, secondary arrays often are designed to detect more different polymorphic sites than primary arrays. For example, a primary array typically detects 1-100 polymorphic sites in 1-100 references. A secondary array can easily analyze 1,000, 10,000 or 100,000 polymorphic sites in reference sequences dispersed throughout the human genome.

The design of suitable probe arrays for analysis of predetermined polymorphisms and interpretation of the hybridization patterns is described in detail in WO 95/11995; EP 717,113; and WO 97/29212. Such arrays typically contain first and second groups of probes which are designed to be complementary to different allelic forms of the polymorphism. Each group contains a first set of probes, which is subdivided into subsets, one subset for each polymorphism. Each subset contains probes that span a polymorphism and proximate bases and are complementary to one allelic form of the polymorphism. Thus, within the first and second probe groups there are corresponding subsets of probes for each polymorphism. The hybridization patterns of these probes to target samples can be analyzed by footprinting or cluster analysis, as described above. For example, if the first and second probes groups contain subsets of probes respectively complementarity to first and second allelic forms of a polymorphic site spanned by the probes, then on hybridization of the array to a sample that is homozygous for the first allelic form all probes in the subset from the first group show specific hybridization, whereas probes in the subset from the second group that span the polymorphism show only mismatch hybridization. The mismatch hybridization is manifested as a footprint of probe intensities in a plot of normalized probe intensity (i.e., target/reference intensity ratio) for the subset of probes in the second group. Conversely, if the target sample is homozygous for the second allelic form, a footprint is observed in the normalized hybridization intensities of probes in the subset from the first probe group. If the target sample is heterozygous for both allelic forms then a footprint is seen in normalized probe intensities from subsets in both probe groups although the depression of intensity ratio within the footprint is less marked than in footprints observed with homozygous alleles.

Alternatively, the first and second groups of probes can contain first, second, third and fourth probe sets. Each of the probe sets can be subdivided into subsets, one for each polymorphism to be analyzed by the array. The first set of probes in each group is spans a polymorphic site and proximate bases and is complementary to one allelic form of the site. The second, third and fourth sets, each have a corresponding probe for each probe in the first probe set, which is identical to a corresponding probe from the first probe set except at the interrogation position, which occurs in the same position in each of the four corresponding probes from the four probe sets, and is occupied by a different nucleotide in the four probe sets.

Such arrays are interpreted in similar manner to the primary arrays having four sets of probes described above. For example, consider a secondary array having first and second groups of probes, each having four sets of probes designed based on first and second allelic forms of a single polymorphic site hybridized to a target containing homozygous first allele. The probes from the first probe set of the first group all show perfect hybridization to the target sample, and probes from other probe sets in the first group all show mismatch hybridization. All probes from the second group of probes show at least one mismatch except one of the four corresponding probes having an interrogation position aligned with the polymorphic site. A probe from the second, third or fourth probes sets probes having an interrogation position occupied by a base that is the complement of the corresponding base in the first allelic form shows specific hybridization.

If such an array is hybridized to a target sample containing homozygous second allelic form, the mirror image hybridization pattern is observed. That is all probes in the first probe set of the second group show matched hybridization, and probes from the second, third and fourth probe sets in the second probe group show mismatch hybridization. All but one probe in the first group of probes shows mismatch hybridization. The one probe showing perfect hybridization has an interrogation site aligned with the polymorphic site and occupied by the complement of the base occupying the polymorphic site in the second allelic form.

If such an array is hybridized to a target sample containing heterozygous first and second allelic forms, the aggregate of the above two hybridization patterns is observed. That is, all probes in the first probe set from both the first and second group show perfect hybridization (albeit with reduced intensity relative to a homozygous target), and one additional probe from the second, third or fourth probe set in each group shows perfect hybridization. In each group, this probe has an interrogation position aligned with the polymorphic site and occupied by a base occupying the polymorphic site in one or other of the allelic forms.

Typically, secondary arrays contain multiple subsets of each of the probe sets described, with a separate subset for each polymorphism. Thus, for example, a secondary array for analyzing a thousand polymorphisms might contain first and second groups of probes, each containing four probe sets, with each of the four probe sets, being divided into 1000 subsets corresponding to the 1000 different polymorphisms. In this situation, analysis of the hybridization patterns from four subsets relating to any given polymorphisms is independent of any other polymorphism.

Analysis of the hybridization pattern of a secondary array to a target sample indicates which polymorphic form is present at some or all of the polymorphic sites represented on an array. Thus, the individual is characterized with a polymorphic profile representing allelic variants present at a substantial collection of polymorphic sites.

## 6. Synthesis and Scanning of Probe Arrays

Arrays of probe immobilized on supports can be synthesized by various methods, A preferred methods is VLSIPS™ (see Fodor et al., 1991, Fodor et al., 1993, *Nature* 364, 555-556; McGall et al., USSN 08/445,332; US 5,143,854; EP 476,014), which entails the use of light to direct the synthesis of oligonucleotide probes in high-density, miniaturized arrays. Algorithms for design of masks to reduce the number of synthesis cycles are described by Hubbel et al., US 5,571,639 and US 5,593,839. Arrays can also be synthesized in a combinatorial fashion by delivering monomers to cells of a support by mechanically constrained flowpaths. See Winkler et al., EP 624,059. Arrays can also be synthesized by spotting monomers reagents on to a support using an ink jet printer. *See id.*; Pease et al,, EP 728,520.

After hybridization of control and target samples to an array containing one or more probe sets as described above and optional washing to remove unbound and nonspecifically bound probe, the hybridization intensity for the respective samples is determined for each probe in the array. For fluorescent labels, hybridization intensity can be determined by, for example, a scanning confocal microscope in photon counting mode. Appropriate scanning devices are described by e.g., Trulson et al., US 5,578,832; Stern et al., US 5,631,734.

## 7. Variations

### (a) Tertiary Arrays for Analysis of RNA

If a secondary array of probes representing a large collection of polymorphic sites is hybridized to an unamplified RNA target sample, then only probes spanning polymorphic sites present in highly expressed RNAs typically hybridize to a detectable extent. A tertiary array is then produced for future use containing only the subsets of probes spanning polymorphic sites represented in highly expressed RNA transcripts. Such an array can be used for allelic profiling without the need to amplify nucleic acids in target sample.

### (b) Distinguishing sequencing errors from polymorphisms in published sequences

A variation on the previously described methods for de novo identification for polymorphisms starts by comparison of two published versions of the same genetic sequences. Frequently, published versions from independent sources show divergence at one or more sites and it is not clear whether the divergence results from sequencing error or is the result of allelic variation. These possibilities can be distinguished by using probe arrays spanning and complementary to one or both of the reported sequences about the site of potential variation. Arrays of either the primary or secondary type described above can be used. The probe arrays are hybridized to target samples from a collection of individuals. The target samples are typically prepared by amplification of nucleic acids using primers flanking a fragment including the site of potential variation between published sequences. If the probe arrays show the same hybridization pattern to each of the target samples, it is concluded that the apparent divergence between the sequences is probably due to sequencing error. If the probe arrays show at least two different hybridization patterns to two different target samples from different individuals, then the site of divergence is confirmed as a bona fide polymorphic site.

In some instances, the original data from which a published sequence was derived is also published or otherwise publicly available. Such data may exist as a sequencing gel ladder or as automatic sequencer trace profiles. Where at least two sources of independent data are available for what purports to be the same sequence, direct comparison of the original data may indicate potential points of divergence that are not represented in the published sequences. Such points of potential divergence are then confirmed or otherwise by hybridizing multiple target samples to suitable arrays as described above.

## 8. Uses of Polymorphic Profiles

After determining a polymorphic profile of an individual or population of individuals, this information can be used in a number of methods.

### a. Association Studies and Diagnosis

The polymorphic profile of an individual may contribute to phenotype of the individual in different ways. Some polymorphisms occur within a protein coding sequence and contribute to phenotype by affecting protein structure. The effect may be neutral, beneficial or detrimental, or both beneficial and detrimental, depending on the circumstances. For example, a heterozygous sickle cell mutation confers resistance to malaria, but a homozygous sickle cell mutation is usually lethal. Other polymorphisms occur in noncoding regions but may exert phenotypic effects indirectly via influence on replication, transcription, and translation. A single polymorphism may affect more than one phenotypic trait. Likewise, a single phenotypic trait may be affected by polymorphisms in different genes. Further, some polymorphisms predispose an individual to a distinct mutation that is causally related to a certain phenotype.

Phenotypic traits include diseases that have known but hitherto unmapped genetic components (e.g., agammaglobulimenia, diabetes insipidus, Lesch-Nyhan syndrome, muscular dystrophy, Wiskott-Aldrich syndrome, Fabry's disease, familial hypercholesterolemia, polycystic kidney disease, hereditary spherocytosis, von Willebrand's disease, tuberous sclerosis, hereditary hemorrhagic telangiectasia, familial colonic polyposis, Ehlers-Danlos syndrome, osteogenesis imperfecta, and acute intermittent porphyria). Phenotypic traits also include symptoms of, or susceptibility to, multifactorial diseases of which a component is, or may be, genetic, such as autoimmune diseases, inflammation, cancer, diseases of the nervous system, and infection by pathogenic microorganisms. Some examples of autoimmune diseases include rheumatoid arthritis, multiple sclerosis, diabetes (insulin-dependent and non-independent), systemic lupus erythematosus and Graves disease. Some examples of cancers include cancers of the bladder, brain, breast, colon, esophagus, kidney, leukemia, liver, lung, oral cavity, ovary, pancreas, prostate, skin, stomach and uterus. Phenotypic traits also include characteristics such as longevity, appearance (e.g., baldness, obesity) , strength, speed, endurance, fertility, and susceptibility or receptivity to particular drugs or therapeutic treatments.

Correlation is performed for a population of individuals who have been tested for the presence or absence of one or more phenotypic traits of interest and for polymorphic profile. The alleles of each polymorphism in the profile are then reviewed to determine whether the presence or absence of a particular allele is associated with the trait of interest. Correlation can be performed by standard statistical methods such as a $\kappa$-squared test and statistically significant correlations between polymorphic form(s) and phenotypic characteristics are noted. For example, it might be found that the presence of allele A1 at polymorphism A correlates with heart disease. As a further example, it might be found that the combined presence of allele A1 at polymorphism A and allele B1 at polymorphism B correlates with increased risk of cancer.

Such correlations can be exploited in several ways. In the case of a strong correlation between a set of one or more polymorphic forms and a disease for which treatment is available, detection of the polymorphic form set in a human or animal patient may justify immediate administration of treatment, or at least the institution of regular monitoring of the patient. Detection of a polymorphic form(s) correlated with serious disease in a couple contemplating a family may also be valuable to the couple in their reproductive decisions. For example, the female partner might elect to undergo in vitro fertilization to avoid the possibility of transmitting such a polymorphism from her husband to her offspring. In the case of a weaker, but still statistically significant correlation between a polymorphic set and human disease, immediate therapeutic intervention or monitoring may not be justified. Nevertheless, the patient can be motivated to begin simple life-style changes (e.g., diet, exercise) that can be accomplished at little cost to the patient but confer potential benefits in reducing the risk of conditions to which the patient may have increased susceptibility by virtue of variant alleles. Identification of a polymorphic profiles in a patient correlated with enhanced receptiveness to one of several treatment regimes for a disease indicates that this treatment regime should be followed.

For animals and plants, correlations between polymorphic profiles and phenotype are useful for breeding for desired characteristics. For example, Beitz et al., US 5,292,639 discuss use of bovine mitochondrial polymorphisms in a breeding program to improve milk production in cows. To evaluate the effect of mtDNA D-loop sequence polymorphism on milk production, each cow was assigned a value of 1 if variant or 0 if wildtype with respect to a prototypical mitochondrial DNA sequence at each of 17 locations considered. Each production trait was analyzed individually with the following animal model:

$Y_{ijkpn} = \mu + YS_i + P_j + X_k + \beta_1 + \ldots \beta_{17} + PE_n + a_n + e_p$ where $Y_{ijknp}$ is the milk, fat, fat percentage, SNF, SNF percentage, energy concentration, or lactation energy record; $\mu$ is an overall mean; $YS_i$ is the effect common to all cows calving in year-season; $X_k$ is the effect common to cows in either the high or average selection line; $\beta_1$ to $\beta_{17}$ are the binomial regressions of production record on mtDNA D-loop sequence polymorphisms; $PE_n$ is permanent environmental effect common to all records of cow n; $a_n$ is effect of animal n and is composed of the additive genetic contribution of sire and dam breeding values and a Mendelian sampling effect; and $e_p$ is a random residual. It was found that eleven of seventeen polymorphisms tested influenced at least one production trait. Bovines having the best polymorphic forms for milk production at these eleven loci are used as parents for breeding the next generation of the herd.

### b. Forensics

Determination of which polymorphic forms occupy a set of polymorphic sites in an individual identifies a set of polymorphic forms that distinguishes the individual. *See generally* National Research Council, *The Evaluation of Forensic DNA Evidence* (Eds. Pollard et al., National Academy Press, DC, 1996). The more sites that are analyzed the lower the probability that the set of polymorphic forms in one individual is the same as that in an unrelated individual.

The capacity to identify a distinguishing or unique set of forensic markers in an individual is useful for forensic analysis. For example, one can determine whether a blood sample from a suspect matches a blood or other tissue sample from a crime scene by determining whether the set of polymorphic forms occupying selected polymorphic sites is the same in the suspect and the sample. If the set of polymorphic markers does not match between a suspect and a sample, it can be concluded (barring experimental error) that the suspect was not the source of the sample. If the set of markers does match, one can conclude that the DNA from the suspect is consistent with that found at the crime scene. If frequencies of the polymorphic forms at the loci tested have been determined (e.g., by analysis of a suitable population of individuals), one can perform a statistical analysis to determine the probability that a match of suspect and crime scene sample would occur by chance.

p(ID) is the probability that two random individuals have the same polymorphic or allelic form at a given polymorphic site. In diallelic loci, four genotypes are possible: AA, AB, BA, and BB. If alleles A and B occur in a haploid genome of the organism with frequencies x and y, the probability of each genotype in a diploid organism are (see WO 95/12607):

Homozygote: $p(AA) = x^2$
Homozygote: $p(BB) = y^2 = (1-x)^2$
Single Heterozygote: $p(AB) = p(BA) = xy = x(1-x)$
Both Heterozygotes: $p(AB+BA) = 2xy = 2x(1-x)$

The probability of identity at one locus (i.e, the probability that two individuals, picked at random from a population will have identical polymorphic forms at a given locus) is given by the equation:

$$p(ID) = (x^2)^2 + (2xy)^2 + (y^2)^2.$$

These calculations can be extended for any number of polymorphic forms at a given locus. For example, the probability of identity p(ID) for a 3-allele system where the alleles have the frequencies in the population of x, y and z, respectively, is equal to the sum of the squares of the genotype frequencies:

$$p(ID) = x^4 + (2xy)^2 + (2yz)^2 + (2xz)^2 + z^4 + y^4$$

In a locus of n alleles, the appropriate binomial expansion is used to calculate p(ID) and p(exc).

The cumulative probability of identity (cum p(ID)) for each of multiple unlinked loci is determined by multiplying the probabilities provided by each locus.

$$\text{cum } p(ID) = p(ID1)p(ID2)p(ID3)\dots p(IDn)$$

The cumulative probability of non-identity for n loci (i.e. the probability that two random individuals will be different at 1 or more loci) is given by the equation:

$$\text{cum } p(nonID) = 1\text{-cum } p(ID).$$

If several polymorphic loci are tested, the cumulative probability of non-identity for random individuals becomes very high (e.g., one billion to one). Such probabilities can be taken into account together with other evidence in determining the guilt or innocence of the suspect.

B. Paternity Testing

The object of paternity testing is usually to determine whether a male is the father of a child. In most cases, the mother of the child is known and thus, the mother's contribution to the child's genotype can be traced. Paternity testing investigates whether the part of the child's genotype not attributable to the mother is consistent with that of the putative father. Paternity testing can be performed by analyzing sets of polymorphisms in the putative father and the child.

If the set of polymorphisms in the child attributable to the father does not match the putative father, it can be concluded, barring experimental error, that the putative father is not the real father. If the set of polymorphisms in the child attributable to the father does match the set of polymorphisms of the putative father, a statistical calculation can be performed to determine the probability of coincidental match.

The probability of parentage exclusion (representing the probability that a random male will have a polymorphic form at a given polymorphic site that makes him incompatible as the father) is given by the equation (see WO 95/12607):

$$p(exc) = xy(1\text{-}xy)$$

where x and y are the population frequencies of alleles A and B of a diallelic polymorphic site.

(At a triallelic site $p(exc) = xy(1\text{-}xy) + yz(1\text{-} yz) + xz(1\text{-}xz) + 3xyz(1\text{-}xyz)))$, where x, y and z and the respective population frequencies of alleles A, B and C).

The probability of non-exclusion is

$$p(non\text{-}exc) = 1\text{-}p(exc)$$

The cumulative probability of non-exclusion (representing the value obtained when n loci are used) is thus:

$$\text{cum } p(non\text{-}exc) = p(non\text{-}exc1)p(non\text{-}exc2)p(non\text{-}exc3)\dots p(non\text{-}excn)$$

The cumulative probability of exclusion for n loci (representing the probability that a random male will be excluded)

$$\text{cum } p(exc) = 1 \text{ - cum } p(non\text{-}exc).$$

If several polymorphic loci are included in the analysis, the cumulative probability of exclusion of a random male is very high. This probability can be taken into account in assessing the liability of a putative father whose polymorphic marker set matches the child's polymorphic marker set attributable to his/her father.

All publications and patent applications cited above are incorporated by reference in their entirety for all purposes to the same extent as if each individual publication or patent application were specifically and individually indicated to be so incorporated by reference. Although the present invention has been described in some detail by way of illustration and example for purposes of clarity and understanding, it will be apparent that certain changes and modifications may be practiced within the scope of the appended claims.

**Claims**

1. A method of polymorphism analysis, comprising:

   (a) constructing a first array of probes spanning and complementary to one or more known DNA sequences;
   (b) hybridizing the first array of probes with nucleic acid samples from different individuals, whereby differences in the hybridization pattern of the samples to the probes among the different individuals indicate the location of one or more polymorphic sites in the one or more DNA sequences;
   (c) repeating (a) and (b) as needed until a collection of polymorphic sites in known DNA sequences has been identified;
   (d) constructing a second array of probes comprising a first set of probes spanning each of the polymorphic sites in the collection and complementary to polymorphic forms present in the known sequences, and a second set of probes spanning each of the polymorphic sites in the collection and complementary to polymorphic forms absent in the known DNA sequences;
   (e) hybridizing the second array of probes to a nucleic acid sample from a further individual, and analyzing the hybridization intensities of probes in the first and second sets of probes to determine a profile of polymorphic forms present in the further individual.

2. The method of claim 1, wherein the further individual in step (e) has a known characteristic whose presence or absence is unknown in the different individuals in step (b).

3. The method of claim 1, further comprising retrieving the known DNA sequences from a computer database whereby the probes spanning the known DNA sequences are selected.

4. The method of claim 3, wherein the known DNA sequences are of unknown function.

5. The method of claim 3, wherein the known DNA sequences are expressed sequence tags.

6. The method of claim 2, wherein the known characteristic is the presence of a disease.

7. The method of claim 2, wherein the known characteristic is the absence of a disease.

8. The method of claim 2, wherein the known characteristic is being suspected of perpetrating a crime.

9. The method of claim 1, wherein the known DNA sequences comprise at least 100 sequences.

10. The method of claim 9, wherein the known sequences occur on at least 2 chromosomes.

11. The method of claim 9, wherein the known sequences occur on each of the 23 human chromosomes.

12. The method of claim 9, wherein at least 10 of the known sequences are unlinked.

13. The method of claim 1, wherein the hybridization pattern of a sample from one of the individuals shows a footprint in which probes spanning a polymorphism hybridize with reduced hybridization intensity relative to the hybridization pattern of a sample from another individual.

14. The method of claim 1, wherein there are at least 10 different individuals in step (b).

15. The method of claim 1, wherein the further nucleic acid sample is RNA or cDNA.

16. The method of claim 15, further comprising determining from the hybridization intensities of probes in the second array a subset of polymorphic sites in a subset of known sequences which are expressed in the further nucleic acid sample.

17. The method of claim 16, further comprising:

   (f) constructing a third array of probes comprising first subset of probes spanning each of the subset of polymorphic sites in the collection and complementary to polymorphic forms present in the subset of known sequences, and a second set of probes spanning each of the subset of polymorphic sites in the collection and complementary to polymorphic forms absent in the subset of known DNA sequences; and

(g) hybridizing the third array of probes to an RNA sample from a third individual, and analyzing the hybridization intensities of probes in the first and second sets of probes to determine a profile of polymorphic forms present in the third individual.

18. The method of claim 17, wherein the RNA sample is obtained without amplification.

19. A method of polymorphism analysis, comprising:

retrieving multiple versions of a nucleic acid sequence from a published source;
comparing the multiple versions to identify point(s) of diversion;
designing an array comprising probes spanning and complementary to part(s) of the nucleic acid sequence spanning the point(s) of diversion; and
hybridizing nucleic acid samples from multiple individuals to the array, whereby the existence of difference(s) in the hybridization intensity of probes spanning a point of diversion among the individuals indicates a polymorphism at the point of diversion, and lack of difference in hybridization intensity of probes spanning a point of diversion among the individuals indicates the point of diversion was due to a sequencing error.

20. The method of claim 19, wherein the published source is a computer database.

21. The method of claim 19, wherein the multiple versions of the nucleic acid sequence are retrieved as trace profiles.

22. A method of polymorphism analysis comprising:

providing an immobilized array of probes comprising a first set of probes spanning each of a collection of polymorphic sites in known sequences and complementary to a first allelic forms of the sites, and a second set of probes spanning each of the polymorphic sites in the collection and complementary to second allelic forms of the sites, wherein the collection of polymorphic sites includes at least 10 unlinked polymorphic sites;
hybridizing a nucleic acid sample from an individual to the array of probes and analyzing the hybridization intensities of probes in the first and second probe sets to determine a profile of polymorphic forms present in the individual.

23. The method of claim 22, wherein the collection of polymorphic sites includes a polymorphic site on each of the 23 human chromosomes.

24. The method of claim 22, wherein the collection of polymorphic sites includes at least 100 polymorphic sites.

25. The method of claim 22, wherein the hybridizing step is repeated for nucleic acid samples from a population of individuals, each of whom is characterized for the presence or absence of a phenotype, to determine a profile of polymorphic forms present in each individual in the population, and the method further comprises correlating profiles of polymorphic forms with the presence or absence of the phenotype in the population.

26. The method of claim 22, wherein the nucleic acid sample is RNA or cDNA.

27. The method of claim 26, further comprising enriching for transcripts of the known sequences in the nucleic acid sample by contacting the nucleic acid sample with probes complementary to the transcripts, whereby the probes hybridize to the transcripts to form complexes, isolating the complexes and dissociating the transcripts of the known sequences from the probes.

28. The method of claim 22, wherein the nucleic acid sample is a genomic DNA sample.

29. The method of claim 22, wherein the nucleic acid sample is prepared by amplification with random primers.

European Patent
Office

**EUROPEAN SEARCH REPORT**

Application Number

EP 99 25 0122

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP 0 785 280 A (AFFYMETRIX INC) 23 July 1997 (1997-07-23) * the whole document * --- | 1-29 | C12Q1/68 |
| X | CHEE M ET AL: "ACCESSING GENETIC INFORMATION WITH HIGH-DENSITY DNA ARRAYS" SCIENCE, vol. 274, 25 October 1996 (1996-10-25), pages 610-614, XP002050640 ISSN: 0036-8075 * the whole document * --- | 1-29 | |
| X | FAN J ET AL: "Genetic mapping: Finding and analyzing single-nucleotide polymorphisms with high-density DNA arrays" AMERICAN JOURNAL OF HUMAN GENETICS, vol. 61, no. 4, SUPPL, 1 October 1997 (1997-10-01), page 1601 XP002089397 ISSN: 0002-9297 * the whole document * --- | 1-29 | TECHNICAL FIELDS SEARCHED (Int.Cl.6) C12Q |
| D,X | WO 97 29212 A (GINGERAS THOMAS A ;CHEE MARK S (US); STRYER LUBERT (US); AFFYMETRI) 14 August 1997 (1997-08-14) * the whole document * --- | 1-29 | |
| D,X | WO 95 11995 A (AFFYMAX TECH NV ;FODOR STEPHEN P A (US); GINGERAS THOMAS R (US); L) 4 May 1995 (1995-05-04) * the whole document * --- | 1-29 | |
| D,X | EP 0 717 113 A (AFFYMAX TECH NV) 19 June 1996 (1996-06-19) * the whole document * --- | 1-29 | |

-/--

The present search report has been drawn up for all claims

| Place of search | Date of completion of the search | Examiner |
|---|---|---|
| THE HAGUE | 27 July 1999 | Hagenmaier, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

14

**European Patent Office**

**EUROPEAN SEARCH REPORT**

Application Number

EP 99 25 0122

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | LIPSHUTZ R J ET AL: "USING OLIGNUCLEOTIDE PROBE ARRAYS TO ACCESS GENETIC DIVERSITY" BIOTECHNIQUES, vol. 19, no. 3, 1995, pages 442-447, XP002916031 ISSN: 0736-6205 * the whole document * | | |
| A | RISCH N ET AL: "THE FUTURE OF GENETIC STUDIES OF COMPLEX HUMAN DISEASES" SCIENCE, vol. 273, 13 September 1996 (1996-09-13), page 1516/1517 XP002050644 ISSN: 0036-8075 * the whole document * | | |
| A | COLLINS ET AL.: "VARIATION ON A THEME: CATALOGING HUMAN SEQUENCE VARIATION" SCIENCE, vol. 278, November 1997 (1997-11), pages 1580-1581, XP002110450 * the whole document * | | TECHNICAL FIELDS SEARCHED (Int.Cl.6) |
| P,X | WANG D G ET AL: "Large-scale identification, mapping, and genotyping of single -nucleotide polymorphisms in the human genome" SCIENCE, vol. 280, 15 May 1998 (1998-05-15), pages 1077-1082, XP002089398 ISSN: 0036-8075 * the whole document * | 1-29 | |

-/--

The present search report has been drawn up for all claims

| Place of search | Date of completion of the search | Examiner |
|---|---|---|
| THE HAGUE | 27 July 1999 | Hagenmaier, S |

EPO FORM 1503 03.82 (P04C01)

**European Patent Office**

**EUROPEAN SEARCH REPORT**

Application Number

EP 99 25 0122

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,X | SAPOLSKY R J ET AL: "High-throughput polymorphism screening and genotyping with high -density oligonucleotide arrays" GENETIC ANALYSIS: BIOMOLECULAR ENGINEERING, vol. 14, no. 5-6, February 1999 (1999-02), page 187-192 XP004158703 ISSN: 1050-3862 * the whole document * | 1-29 | |
| | | | TECHNICAL FIELDS SEARCHED (Int.Cl.6) |

The present search report has been drawn up for all claims

| Place of search | Date of completion of the search | Examiner |
|---|---|---|
| THE HAGUE | 27 July 1999 | Hagenmaier, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)

**ANNEX TO THE EUROPEAN SEARCH REPORT
ON EUROPEAN PATENT APPLICATION NO.**

EP 99 25 0122

This annex lists the patent family members relating to the patent documents cited in the above-mentioned European search report.
The members are as contained in the European Patent Office EDP file on
The European Patent Office is in no way liable for these particulars which are merely given for the purpose of information.

27-07-1999

| Patent document cited in search report | | Publication date | Patent family member(s) | | Publication date |
|---|---|---|---|---|---|
| EP 0785280 | A | 23-07-1997 | US | 5858659 A | 12-01-1999 |
| WO 9729212 | A | 14-08-1997 | AU | 2189397 A | 28-08-1997 |
| WO 9511995 | A | 04-05-1995 | AU | 8126694 A | 22-05-1995 |
| | | | EP | 0730663 A | 11-09-1996 |
| | | | JP | 9507121 T | 22-07-1997 |
| | | | US | 5861242 A | 19-01-1999 |
| | | | US | 5837832 A | 17-11-1998 |
| EP 0717113 | A | 19-06-1996 | US | 5795716 A | 18-08-1998 |

EPO FORM P0459

For more details about this annex : see Official Journal of the European Patent Office, No. 12/82